# EUROPÄISCHE PATENTSCHRIFT

(11) **EP 1 530 717 B1**
(45) Veröffentlichungstag und Bekanntmachung des Hinweises auf die Patenterteilung: **28.11.2007**
(21) Anmeldenummer: 03787720.6
(22) Anmeldetag: 23.07.2003
(51) Int. Cl.: G01N 33/00, F24F 3/16

(54) **ZWANGSBELÜFTETE MESSEINRICHTUNG ZUR ERFASSUNG LUFTTECHNISCHER PARAMETER**
FORCED-VENTILATED MEASURING DEVICE FOR DETECTING TECHNICAL PARAMETERS RELATING TO THE AIR
DISPOSITIF DE MESURE A VENTILATION FORCEE POUR LA DETECTION DE PARAMETRES TECHNIQUES RELATIFS A L'AIR

(30) Priorität: 25.07.2002 DE 10233755
(43) Veröffentlichungstag der Anmeldung: 18.05.2005
(73) Patentinhaber: T.E.M. TECHNISCHE ENTWICKLUNGEN UND MANAGEMENT GMBH, 63840 Hausen (DE)
(72) Erfinder: RUMP, Hanns, 63840 Hausen (DE)
(74) Vertreter: Mierswa, Klaus
(86) Internationale Anmeldenummer: PCT/DE2003/002473
(87) Internationale Veröffentlichungsnummer: WO 2004/017065

(56) Entgegenhaltungen:
- DE-A- 10 021 067
- DE-A- 19 842 068
- DE-A- 19 900 306
- US-A- 4 606 219
- PATENT ABSTRACTS OF JAPAN vol. 009, no. 139 (M-387), 14. Juni 1985 (1985-06-14) & JP 60 020027 A (MITSUBISHI JUKOGYO KK), 1. Februar 1985 (1985-02-01)

## Beschreibung

### Technisches Gebiet:

Die Erfindung betrifft einen Apparat zur elektronischen Messung lufttechnischer Größen wie Lufttemperatur, Luftfeuchte und Luftqualität oder Gaskonzentration.

### Stand der Technik:

Die lufttechnischen Parameter wie Luftfeuchte, Lufttemperatur, Luftqualität oder Gaskonzentrationen sind Größen, welche für die Regelung / Steuerung raumlufttechnischer Anlagen (RTA) benötigt werden und aus diesem Grunde technisch erfasst bzw. gemessen werden müssen.

Luftfeuchte wird zumeist als relative Feuchte, % r.F., angegeben und beschreibt den Sättigungsgrad der Luft mit Wasser bei gegebener Temperatur.

Ein geeignetes kostengünstiges Messprinzip sind zum Beispiel beidseitig metallbedampfte Polymere, welche einen elektrischen Kondensator bilden. Die elektrisch zu messende Kapazität des Kondensators ist eine Funktion der Wasseraufnahme des Polymers, welche in einem Gleichgewichtszustand unter Einfluß der Luftfeuchte und der Umgebungstemperatur ist.

Lufttemperatur wird in "Grad Celsius" angegeben, seltener in der physikalischen Notation "Grad Kelvin". Kostengünstige Messprinzipen nutzen den Einfluss der Temperatur auf elektrisch leitende Materialien, etwa auf einen Platinleiter. Es wird der Ohmsche Widerstand gemessen.

Luftqualität wird durch den Anteil organischer Substanzen, nämlich VOC = volatile organic compounds, in der Luft angegeben und zwar in mg/m3. VOC sind zumeist auch geruchstragend und entstehen als Stoffwechselnebenprodukte bei Anwesenheit von Menschen und / oder als Ausdünstungen aus Kunststoffen, Materialien oder sonstigen chemischen Prozessen, wie Tabakrauch, Küchendämpfe, etc.

Kostengünstige Messprinzipien nutzen breitbandig detektierende Metalloxid-Halbleifersensoren, welche alle oxidierbaren Luftbestandteile detektieren können. Eingebettet in komplexen Signalauswertungen sind kostengünstige, stabile und datentreue Messsensoren bekannt, etwa aus der DE 195 43 296 5 oder der EP 96 118 420.

Detektoren zur Messung des von Menschen abgegebenen Kohlendioxid, CO₂, sind ebenfalls bekannt; sie erfassen jedoch in nachteiliger Weise nicht die Anteile anderer organischer und gelegentlich toxischer Luftbestandteile, etwa Formaldehyd.

Messgeräte zur Erfassung von einer oder mehrerer raumlufttechnischer Größen müssen sowohl in Räumen, Fahrzeugkabinen als auch in Luftkanälen installiert werden.

Bekannt ist weiter, dass sogenannte Ortseinflüsse, zum Beispiel die Messung der Lufttemperatur und in besonderem Maße die der Luftfeuchte, erheblich verfälschen. Darunter sind solche Einflüsse zu verstehen, wie etwa Wärmestrahlung / Infrarot-Strahlung oder Wärme / Kältebrücken bei Montagebrücken bzw. zur Wand, auf der das Messgerät montiert ist.

Bekannt ist von hochwertigen klimatechnischen Messgeräten, die Detektoren gegen Wärme- oder Kältebrücken thermisch sorgfältig zu isolieren und gegen thermische Strahlung abzuschirmen und die Detektoren künstlich mit Hilfe eines Ventilators zu belüften. Im Ergebnis werden damit Ortseinflüsse vermieden und die Präzision der Messung der tatsächlichen Luftparameter wird erhöht.

Nachteilig ist hingegen, dass diese Messgeräte voluminös und sehr teuer sind und permanent das Geräusch des Ventilators zu hören ist. Für Anwendungen in Wohnraumumgebung oder in Automobilen sind diese Geräte daher nicht geeignet.

Sensoren zur Erfassung der Konzentration von Gasen in Luft haben gelegentlich das Problem, dass das Gasgemisch nicht homogen ist und es zu lokalen Konzentrationsan- oder -abreicherungen kommen kann.

Durch die DE 100 21 067 A ist wenigstens ein elektromotorisch angetriebener Lüfter zum Belüften eines Sensors bekannt geworden, der beispielsweise Bestandteil einer geregelten Fahrzeugheizanlage ist, wobei der Lüfter ein Lüftergehäuse mit einem Einlass und einem Auslass aufweist. Innerhalb des Lüftergehäuses ist ein Lüfterrad antreibbar von einem bürstenlosen elektronisch kommutierten Elektromotor. Der Sensor, der beispielsweise ein Temperatursensor ist, ist beim oder im Einlass des Lüftergehäuses angeordnet, so dass bei angetriebenem Lüfterrad der Sensor mit der Luft belüftet wird, die sich das Lüftergebläse aus der Umgebung ansaugt. Das Belüften des Sensors in dieser Weise wird durchgeführt, damit ein Regler der Fahrzeugheizanlage schnell den zu ermittelnden Istwert angezeigt bekommt. Es wird hier vorgeschlagen, einen Induktionsmotor einzubauen, dessen antreibendes Drehfeld durch Fremdkommutierung erzwungen wird. Hierbei wird ausgenützt, dass unabhängig von der Stellung des jeweiligen Rotors des Induktionsmotors beim Einschalten ein konstruktiv bestimmbares Anlaufmoment zur Verfügung steht.

### Technische Aufgabe:

Aufgabenstellung der Erfindung ist es, ein Gerät zur Erfassung der Luftparameter mindestens einer der nachstehend genannten Größen zu erfassen, nämlich Luftfeuchte, in % rel. Feuchte, oder Lufttemperatur, in Grad Celsius, oder Luftqualität, in VOC-Anteil oder in CO₂-Anteil, oder die Konzentration von Gasen in Luft.

### Lösung der Aufgabe sowie Vorteile der Erfindung:

Die Lösung der Aufgabe besteht bei einem Apparat der eingangs genannten Gattung darin, dass ein oder mehrere Sensoren zur Erfassung einer oder mehrerer lufttechnischer Größen in einem einzigen Gehäuse untergebracht sind, wobei das Gehäuse zur Erzeugung einer Luftströmung durch einen Ventilator belüftet ist, welcher nach dem Prinzip eines geräuschlosen Ionengebläses arbeitet.

Somit wird zur Erzeugung einer Luftströmung erfindungsgemäß vorgeschlagen, ein elektrostatisches Gebläse in Anlehnung an P 198 42 068 zu nutzen. Dazu wird Luft elektrisch etwa an Koronarspitzen ionisiert, wobei die gebildeten Ionen in einem elektrischen Feld auf eine Elektrode, Anode, zu beschleunigt werden und dabei Luftmoleküle aus der Umgebung mitreißen. Die Anode besteht aus einem Gitter oder aus gespannten Drähten, sodass zwar einige der Ionen aufgefangen werden, die beschleunigten Luftmoleküle jedoch über die Anode hinaus weiter in den Raum fliegen. Luftströmungen in der Größenordnung von 13m/sec lassen sich realisieren. Die Luftmenge, Volumen, bestimmt sich aus der Fläche der Anordnung. Ein elektrostatischer Lüfter erzeugt vorteilhaft absolut keine Geräusche.

Kurzbeschreibung der Zeichnung, in der zeigen:
- Figur 1: eine Erläuterung des Funktionsprinzips und
- Figur 2: einen prinzipiellen Aufbau eines erfindungsgemäßes Messgerätes für lufttechnische Messgrößen.

### Wege zur Ausführung der Erfindung:

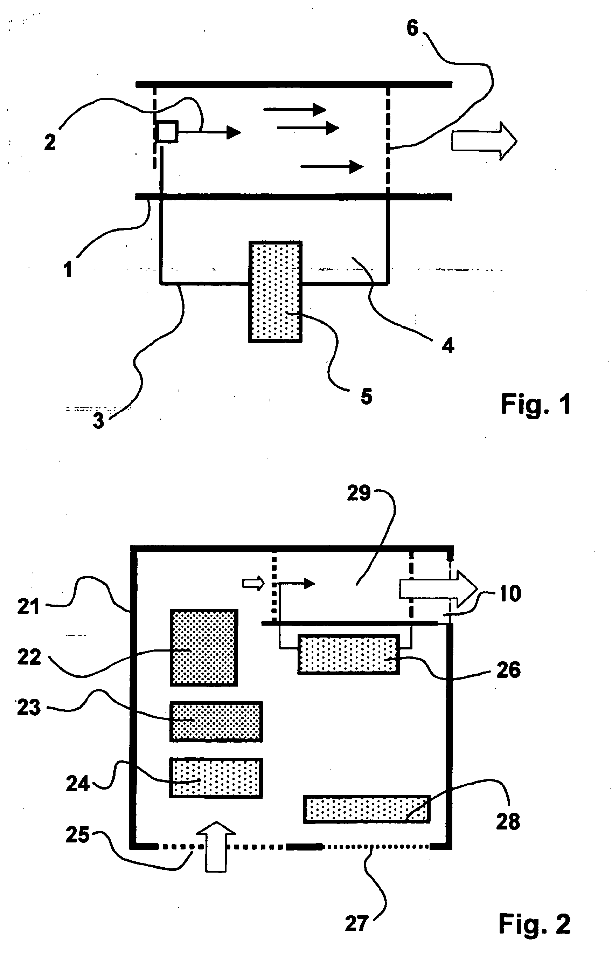

Gemäß der Figur 1 befindet sich in einem Luftkanal 1 auf einem Träger eine möglichst spitze Nadel 2 oder ein Bündel von elektrisch leitenden Spitzen, welche über eine elektrische Leitung 3 an den Minuspol einer hohen elektrischen Gleichspannung angeschlossen sind. Der Pluspol, also die Anode 6, wird aus einem Metallgitter oder aus einer Anordnung gespannter, dünner Drähte gebildet, welche über eine elektrische Leitung 4 an eine Hochspannungsquelle 5 angeschlossen ist. Um die Spitze der Nadel 2 bildet sich ein hohes elektrisches Feld, in welchem Luftmoleküle ionisiert werden. Die Ionen werden in dem Feld, welches sich zwischen der Nadel 2 und der Anode 6 bildet, beschleunigt und reißen dabei Luftmoleküle mit, was eine Luftströmung in Richtung Anode zur Folge hat. Währen die Ionen vom Anodengitter "eingefangen" werden, fliegen die beschleunigten Luftmoleküle aufgrund der innewohnenden kinetischen Energie weiter in den Raum hinaus. Der Wirkungsgrad kann durch eine oder mehrere Beschleunigungsgitter 7 verbessert werden.

Ein lufttechnischer Sensor hat ein inneres Gehäusevolumen von beispielsweise 0,1 ltr. Wird ein Luftaustausch von ca. 5/min angestrebt, muss demnach ein Volumen von 0,51 ltr. / Minute gefördert werden.

Ein elektrostatischer Lüfter mit einer Austrittsfläche von nur 5cm² hat bei einer Austrittsgeschwindigkeit von 2m/sec ein Fördervolumen von 600cm³/min.

Permanente Belüftung des Sensorgehäuses vermindert vorteilhaft den Einfluss von Ortseinflüssen, vor allem solche aus thermischer Strahlung oder durch Kälte oder Wärmebrücken.

Das erfindungsgemäße Messgerät für lufttechnische Messgrößen hat einen prinzipiellen Aufbau, wie er in Figur 2 gezeigt ist; es besteht aus den Bauteilen Gehäuse 21, VOC (Luftgüte-) Sensor 22, Feuchtesensor 23, Temperatursensor 24, Lufteinlass 25, Hochspannungsquelle 26, Kabelanschluss / 2. Lufteinlass 27, Ionengebläse 28, Luftauslass 29.

In einer Variante der Erfindung wird die Hochspannungserzeugung mit Hilfe eines piezoelektrischen Bauteils, ähnlich der DE 101 290 41, erzeugt. Dabei wird durch einen elektrischen Frequenzgenerator das piezoelektrische Bauteil zu Schwingungen angeregt. An anderer Stelle wird eine hohe Wechselspannung abgegriffen, gleichgerichtet und dem Ionengebläse zugeführt.

Speziell für die Erfassung klimatechnischer Größen in den Kabinen von Fahrzeugen aller Art, insbesondere von Automobilen, werden von Ventilatoren zwangsbelüftete Sensoren - insbesondere Temperatursensoren - eingesetzt. Aufgrund der permanenten Geräuschentwicklung werden die Sensoren hinter das Armaturenbrett eingebaut. Es wird insofern die Temperatur etwa in Höhe der Beine der Passagiere gemessen. Sinnvoller wäre es jedoch, die Lufttemperatur etwa in Kopfhöhe zu messen.

Erfindungsgemäß wird vorgeschlagen, mit Ionengebläse künstlich belüftete Sensoren in die Kopfstützen oder in die Dachholme einzusetzen. Über Datenbussysteme können die Sensoren ihre Daten an den zentralen Klimacomputer liefern. Vorteilhaft sind die von Ortseinflüssen befreiten Daten, die erhöhte Ansprechgeschwindigkeit der Sensoren und die absolute Geräuschfreiheit.

Gemeinsam ist allen erfindungsgemäßen Apparaten, dass sie folgende Merkmale haben, nämlich einen oder mehrere Sensoren zur Erfassung klimatechnischer Größen, wie etwa Luftqualität, Luftfeuchte oder Lufttemperatur sowie ein nach dem Prinzip der beschleunigten Luftionen arbeitendes Ionengebläse zum Austausch der Luft innerhalb des Gehäuses.

### Bezugszeichenliste:

Figur 1:
   - 1: Luftkanal
   - 2: Ionisationsspitze, nach dem Koronarprinzip arbeitend
   - 3: Elektrische Anschlussleitung für (-) -Pol, ca. 3000Volt Gleichspannung
   - 4: Elektrische Anschlussleitung für (+)-Pol, ca. 3000Volt Gleichspannung
   - 5: Hochspannungsquelle
   - 6: Gitterförmige Anode
   - 7: Beschleunigungsgitter
Figur 2:
   - 21: Gehäuse
   - 22: VOC (Luftgüte-) Sensor
   - 23, 24: Feuchtesensor und Temperatur-Sensor
   - 25: Lufteinlass
   - 26: Hochspannungs-Quelle
   - 27: Kabelanschluss / 2. Lufteinlass
   - 28: Ionengebläse
   - 29: Luftauslass

## Patentansprüche

1. Apparat zur elektronischen Messung lufttechnischer Größen wie Lufttemperatur, Luftfeuchte und Luftqualität oder Gaskonzentration,
**dadurch gekennzeichnet,**
**dass** ein oder mehrere Sensoren zur Erfassung einer oder mehrerer lufttechnischer Größen in einem einzigen Gehäuse untergebracht sind, wobei das Gehäuse durch einen Ventilator belüftet wird, welcher nach dem Prinzip eines geräuschlosen Ionengebläses arbeitet.

2. Apparat nach Anspruch 1, **dadurch gekennzeichnet,**
**dass** das Ionengebläse ein oder mehrere Beschleunigungsgitter aufweist.

3. Apparat nach Anspruch 1 oder 2, **dadurch gekennzeichnet, dass** sowohl das den oder die Sensoren tragende Gehäuse als auch das Ionengebläse miniaturisiert sind und zusammen eine kompakte, kleine Einheit bilden.

4. Apparat nach einem der Ansprüche 1 bis 3, **dadurch gekennzeichnet, dass** die Hochspannungserzeugung mit Hilfe eines piezoelektrischen Wandlers erfolgt.

5. Apparat nach mindestens einem der Ansprüche 1-4, **dadurch gekennzeichnet, dass** Sensoren des erfindungsgemäßen Prinzips in Automobilen eingesetzt sind.

6. Apparat nach Anspruch 5, **dadurch gekennzeichnet, dass** Sensoren nach Anspruch 1-4 in Kopfstützen der Sitze von Automobilen eingebaut sind.

7. Apparat nach Anspruch 5, **dadurch gekennzeichnet, dass** Sensoren nach Anspruch 1-4 in die Dachholme von Automobilen eingebaut sind.

8. Apparat nach Anspruch 5, **dadurch gekennzeichnet, dass** Sensoren nach Anspruch 1-4 in den Dachhimmel von Automobilen eingebaut sind.

## Claims

1. An apparatus for the electronic measurement of technical parameters relating to the air such as air temperature, air humidity and air quality or gas concentration,
**characterized in that**
one or more sensors for detecting one or more technical air quantities are accommodated in a single housing, whereby the housing is ventilated by a fan that operates according to the principle of a silent ionizing blower.

2. The apparatus according to Claim 1, **characterized in that** the ionizing blower has one or more accelerating grids.

3. The apparatus according to Claim 1 or 2, **characterized in that** the housing that holds the sensor or sensors as well as the ionizing blower are miniaturized and together form a compact, small unit.

4. The apparatus according to any of Claims 1 to 3, **characterized in that** the generation of high-voltage is carried out using a piezoelectric transducer.

5. The apparatus according to at least one of Claims 1 to 4, **characterized in that** sensors having the principle according to the invention are used in automobiles.

6. The apparatus according to Claim 5, **characterized in that** sensors according to Claims 1 to 4 are installed in the headrests of the seats of automobiles.

7. The apparatus according to Claim 5, **characterized in that** sensors according to Claims 1 to 4 are installed in the roof supports of automobiles.

8. The apparatus according to Claim 5, **characterized in that** sensors according to Claims 1 to 4 are installed in the roof liners of automobiles.

## Revendications

1. Appareil pour la mesure électronique de grandeurs techniques relatives à l'air telles que la température de l'air, l'humidité de l'air et la qualité de l'air ou la concentration de gaz,
**caractérisé en ce que**
un ou plusieurs capteurs pour la détection d'une ou de plusieurs grandeurs techniques relatives à l'air sont logés dans un boîtier unique, le boîtier étant ventilé par un ventilateur qui travaille selon le principe d'une soufflerie ionique silencieuse.

2. Appareil selon la revendication 1, **caractérisé en ce que**
la soufflerie ionique présente une ou plusieurs grilles d'accélération.

3. Appareil selon la revendication 1 ou 2, **caractérisé en ce que**
aussi bien le boîtier portant le ou les capteurs que la soufflerie ionique sont miniaturisés et forment ensemble une petite unité compacte.

4. Appareil selon l'une des revendications 1 à 3, **caractérisée en ce que**
la génération de haute tension a lieu à l'aide d'un convertisseur piézoélectrique.

5. Appareil selon au moins l'une des revendications 1 à 4, **caractérisé en ce que**
les capteurs du principe selon l'invention sont mis en oeuvre dans des automobiles.

6. Appareil selon la revendication 5, **caractérisé en ce que**
les capteurs selon les revendications 1 à 4 sont montés dans les appuie-têtes de sièges d'automobiles .

7. Appareil selon la revendication 5, **caractérisé en ce que**
les capteurs selon les revendications 1 à 4 sont montés dans les longerons de pavillon d'automobiles.

8. Appareil selon la revendication 5, **caractérisé en ce que**
les capteurs selon les revendications 1 à 4 sont montés dans les pavillons d'automobiles.
